# EUROPEAN PATENT APPLICATION

(11) **EP 4 751 712 A1**
(43) Date of publication of application: **03.06.2026**
(21) Application number: 24845644.4
(22) Date of filing: 24.07.2024
(51) Int. Cl.: A61K 8/49, A61K 8/34, A61K 8/37, A61K 8/41, A61Q 17/04

(54) **SUNSCREEN COSMETIC**

(30) Priority: 25.07.2023 JP 2023120625
(71) Applicant: Kao Corporation, Tokyo 103-8210 (JP)
(72) Inventor: SHIMADA, Aki, Tokyo 103-8210 (JP)
(74) Representative: Hoffmann Eitle
(86) International application number: PCT/JP2024/026415
(87) International publication number: WO 2025/023269

(57) **Abstract**

A sunscreen cosmetic which has an excellent ultraviolet protection effect, gives favorable feel upon application, and is stable without crystallization. The present invention relates to a sunscreen cosmetic comprising the following components (A), (B), and (C), wherein a mass ratio of a component (A1) to a component (A2), (A1)/(A2), is 3.5 or more and 120 or less, a mass ratio of the component (B) to a total amount of the component (A1) and the component (A2), (B)/(A1)+(A2)), is 2.7 or more and 9 or less, and a mass ratio of a component (B1) to the component (A), (B1)/(A), is less than 1: (A) 10% by mass or more and 20% by mass or less of a water-insoluble organic ultraviolet absorber which is in a solid state at 25°C, the water-insoluble organic ultraviolet absorber comprising (A1) ethylhexyl triazone and (A2) bis-ethylhexyloxyphenol methoxy triazine, (B) 12% by mass or more and 40% by mass or less of an oil agent which is in a liquid state at 25°C, the oil agent comprising (B1) a water-insoluble organic ultraviolet absorber which is in a liquid state at 25°C, and (C) 48% by mass or more and 78% by mass or less of ethanol.

## Description

### Field of the Invention

The present invention relates to a sunscreen cosmetic.

### Background of the Invention

Sunscreen cosmetics are supplemented with ultraviolet absorbers or ultraviolet scattering agents in order to impart a high ultraviolet protection effect. Particularly, sunscreen cosmetics for those who enjoy outdoor activities are required to have a high SPF value, which is typically an index for protecting against sunburn caused by UVB, and also to have a high PA value, which is typically an index for a UVA protection effect (also called UVAPF value). Ethylhexyl triazone and bis-ethylhexyloxyphenol methoxy triazine are used as an ultraviolet absorber exerting such an ultraviolet protection effect (Patent Literature 1). However, these excellent ultraviolet absorbers are in a solid state at room temperature and must therefore be blended with a large amount of a liquid oil for stable formulation of liquid sunscreen cosmetics (Patent Literature 1).

(Patent Literature 1) JP-A-2017-780554

### Summary of the Invention

The present invention relates to a sunscreen cosmetic comprising the following components (A), (B), and (C), wherein a mass ratio of a component (A1) to a component (A2), (A1)/(A2), is 3.5 or more and 120 or less, a mass ratio of the component (B) to a total amount of the component (A1) and the component (A2), (B)/((A1)+(A2)), is 2.7 or more and 9 or less, and a mass ratio of a component (B1) to the component (A), (B1)/(A), is less than 1:
(A) 10% by mass or more and 20% by mass or less of a water-insoluble organic ultraviolet absorber which is in a solid state at 25°C, the water-insoluble organic ultraviolet absorber comprising (A1) ethylhexyl triazone and (A2) bis-ethylhexyloxyphenol methoxy triazine,
(B) 12% by mass or more and 40% by mass or less of an oil agent which is in a liquid state at 25°C, the oil agent comprising (B1) a water-insoluble organic ultraviolet absorber which is in a liquid state at 25°C, and
(C) 48% by mass or more and 78% by mass or less of ethanol.

### Detailed Description of the Invention

The present invention relates to a sunscreen cosmetic which contains a water-insoluble organic ultraviolet absorber which is in a solid state at 25°C, has an excellent ultraviolet protection effect, gives favorable feel upon application, and is stable with suppressed crystallization.

The present inventor found that a composition in which contents of ethylhexyl triazone and bis-ethylhexyloxyphenol methoxy triazine, a content of a liquid oil containing a liquid ultraviolet absorber, and their content ratios are in certain ranges and is further dissolved in ethanol, serves to providing a sunscreen cosmetic which has an excellent ultraviolet protection effect, gives favorable feel upon application, and is stable such that crystallization of the water-insoluble ultraviolet absorbers which are in a solid state at room temperature is suppressed.

The present invention provides a sunscreen cosmetic which contains a water-insoluble ultraviolet absorber which is in a solid state at 25°C, has an excellent ultraviolet protection effect, gives favorable feel upon application, and is stable with suppressed crystallization.

One aspect of the sunscreen cosmetic of the present invention relates to a sunscreen cosmetic comprising the following components (A), (B), and (C):
(A) 10% by mass or more and 20% by mass or less of a water-insoluble organic ultraviolet absorber which is in a solid state at 25°C, the water-insoluble organic ultraviolet absorber comprising (A1) ethylhexyl triazone and (A2) bis-ethylhexyloxyphenol methoxy triazine,
(B) 12% by mass or more and 40% by mass or less of an oil agent which is in a liquid state at 25°C, the oil agent comprising (B1) a water-insoluble organic ultraviolet absorber which is in a liquid state at 25°C, and
(C) 48% by mass or more and 78% by mass or less of ethanol, wherein
   a mass ratio of the component (A1) to the component (A2), (A1)/(A2), is 3.5 or more and 120 or less,
   a mass ratio of the component (B) to a total amount of the component (A1) and the component (A2), (B)/((A1)+(A2)), is 2.7 or more and 9 or less, and
   a mass ratio of the component (B1) to the component (A), (B1)/(A), is less than 1.

The component (A) in the cosmetic of the present invention is a water-insoluble organic ultraviolet absorber which is in a solid state at 25°C, the water-insoluble organic ultraviolet absorber containing (A1) ethylhexyl triazone and (A2) bis-ethylhexyloxyphenol methoxy triazine.

The solid state at 25°C refers to a state having no flowability at 25°C under 1 atm. The water insolubility means that the degree of dissolution in water is 1% by weight or less. The organic ultraviolet absorber is an organic compound, as a matter of course.

The ethylhexyl triazone (also called 2,4,6-tris[4-(2-ethylhexyloxycarbonyl)anilino]-1,3,5-triazine) (A1), which is a triazine-based ultraviolet absorber, is excellent particularly in the ability to absorb ultraviolet ray in a wavelength range of UVB, is in a solid state at 25°C, and is favorably stable. "UVINUL T150" (BASF Japan Ltd.) can be used as a commercially available product of ethylhexyl triazone.

The bis-ethylhexyloxyphenol methoxy triazine (also called 2,4-bis{[4-(2-ethylhexyloxy)-2-hydroxy]phenyl}-6-(4-methoxyphenyl)-1,3,5-triazine) (A2), which is also a triazine-based ultraviolet absorber, is an ultraviolet absorber which absorbs ultraviolet ray in a wide wavelength range from UVA to UVB, is in a solid state at 25°C, and has high light stability. "TINOSORB S" (BASF Japan Ltd.) can be used as a commercially available product of bis-ethylhexyloxyphenol methoxyphenyl triazine.

Examples of the water-insoluble organic ultraviolet absorber which is in a solid state at 25°C as the component (A) include 4-tert-butyl-4'-methoxydibenzoylmethane, diethylamino hydroxybenzoyl hexyl benzoate, drometrizole trisiloxane, ethylhexyl dimethoxybenzylidene dioxoimidazolidine propionate, in addition to the components (A1) and (A2). One of these water-insoluble organic ultraviolet absorbers can be contained alone, or two or more thereof can be contained in combination. Among them, one or two members selected from the group consisting of diethylamino hydroxybenzoyl hexyl benzoate and drometrizole trisiloxane are more preferably used.

Examples of commercially available products of these components include: "PARSOL 1789" (4-tert-butyl-4'-methoxydibenzoylmethane) (manufactured by DSM-Firmenich); "UVINUL APLUS" (diethylamino hydroxybenzoyl hexyl benzoate) and "UVINUL M40" (2-hydroxy-4-methoxybenzophenone); "Mosacare A440" (drometrizole trisiloxane) (manufactured by UFC Corporation); "Soft Shade DH" (ethylhexyl dimethoxybenzylidene dioxoimidazolidine propionate) (manufactured by Ajinomoto Co., Inc.); "SUN-SHEL DMTS" (drometrizole trisiloxane) (manufactured by SERA SOO Co., Ltd.); and "Tinosorb A2B" (trisbiphenyltriazine) (manufactured by BASF SE).

The component (A) is contained at preferably 10% by mass or more, more preferably 10.5% by mass or more, further more preferably 11% by mass or more, in the whole amount of the sunscreen cosmetic of the present invention from the viewpoint of obtaining a sunscreen cosmetic which has an excellent ultraviolet protection effect and is stable without causing crystallization. The component is contained preferably at 20% by mass or less, more preferably 18% by mass or less, further more preferably 16% by mass or less, from the same viewpoint as above. Specifically, the content is preferably 10% by mass or more and 20% by mass or less, more preferably 10.5% by mass or more and 18% by mass or less, further more preferably 11% by mass or more and 16% by mass or less.

The whole amount of the sunscreen cosmetic of the present invention refers to the whole amount of a cosmetic composition, and when the a propellant is contained, the whole amount of the sunscreen refers to the whole amount of a stock solution excluding the propellant.

The content of the component (A1) is preferably 1.5% by mass or more, more preferably 1.8% by mass or more, further more preferably 2% by mass or more, in the whole amount of the sunscreen cosmetic of the present invention from the viewpoint of obtaining a sunscreen cosmetic which has an excellent ultraviolet protection effect and is stable without causing crystallization. The component (A1) is contained at preferably 10% by mass or less, more preferably 9% by mass or less, further more preferably 7% by mass or less, from the same viewpoint as above. Specifically, the content is preferably 1.5% by mass or more and 10% by mass or less, more preferably 1.8% by mass or more and 9% by mass or less, further more preferably 2% by mass or more and 7% by mass or less.

The content of the component (A2) is preferably 0.01% by mass or more, more preferably 0.02% by mass or more, further more preferably 0.05% by mass or more, in the whole amount of the sunscreen cosmetic of the present invention from the viewpoint of obtaining a sunscreen cosmetic which has an excellent ultraviolet protection effect and is stable without causing crystallization. The component (A2) is contained at preferably 1% by mass or less, more preferably 0.9% by mass or less, further more preferably 0.8% by mass or less, from the same viewpoint as above. Specifically, the content is preferably 0.01% by mass or more and 1% by mass or less, more preferably 0.02% by mass or more and 0.9% by mass or less, further more preferably 0.05% by mass or more and 0.8% by mass or less.

A mass ratio of the component (A1) to the component (A2), (A1)/(A2), is preferably 3.5 or more, more preferably more than 5.0, further more preferably 5.5 or more, from the viewpoint of obtaining a sunscreen cosmetic which has an excellent ultraviolet protection effect, gives favorable feel upon application, and is stable without causing crystallization, and is preferably 120 or less, more preferably less than 100, further more preferably 50 or less, even more preferably 10 or less, from the same viewpoint as above. Specifically, the mass ratio is preferably 3.5 or more and 120 or less, more preferably more than 5.0 and less than 100, further more preferably 5.5 or more and 50 or less, even more preferably 3.7 or more and 10 or less.

All of 2-ethylhexyl p-methoxycinnamate, oxybenzone, and octocrylene are components known as ultraviolet absorbers. The total content of these three components in the sunscreen cosmetic of the present invention is preferably 1% by mass or less, more preferably 0.5% by mass or less, further more preferably substantially 0, from the viewpoint that the crystallization of the water-insoluble ultraviolet absorber which is in a solid state at 25°C as the component (A) can be suppressed even if a small amount of the water-insoluble ultraviolet absorber which is in a liquid state at 25°C as the component (B1)is used.

The component (B) is an oil agent which is in a liquid state at 25°C, the oil agent containing (B1) a water-insoluble organic ultraviolet absorber which is in a liquid state at 25°C. The component (B) is used for the purpose of dissolving the component (A) and producing use impression of no stickiness and, in the present invention, also exhibits an effect of improving feel upon application.

The liquid state at 25°C refers to having flowability at 25°C under 1 atm.

The water-insoluble organic ultraviolet absorber (B1) which is in a liquid state at 25°C, contained in the component (B) is preferably one or more members selected from the group consisting of 2-ethoxyethyl p-methoxycinnamate, an isopropyl p-methoxycinnamate-diisopropyl cinnamic acid ester mixture, methyl bis(trimethylsiloxy)silylisopentyl trimethoxycinnamate, diethylamino hydroxybenzoyl hexyl benzoate, amyl p-dimethylaminobenzoate, 2-ethylhexyl p-dimethylaminobenzoate, ethylene glycol salicylate, 2-ethylhexyl salicylate, butyloctyl salicylate, benzyl salicylate, and homomenthyl salicylate. 2-Ethylhexyl salicylate is further more preferably contained therein from the viewpoint of enhancing the ultraviolet protection effect and from the viewpoint of dissolving the component (A).

Examples of commercially available products of these water-insoluble organic ultraviolet absorbers which are in a liquid state include "PARSOL EHS" (2-ethylhexyl salicylate), "PARSOL HMS" (homomenthyl salicylate), "PARSOL SLX" (INCI name: polysilicone-15) (dimethicodiethyl benzalmalonate)) (all manufactured by DSM-Firmenich).

The component (B) contains an oil agent which is in a liquid state at 25°C other than the component (B1). Examples of the oil agent which is in a liquid state other than the component (B1) include one or more oil agents which are in a liquid state at 25°C, the oil agents being selected from the group consisting of an ester oil other than the component (B1), a hydrocarbon oil, an ether oil, a silicone oil, and a fluorine oil. Among them, it is preferred to contain an ester oil other than the component (B1), and it is more preferred to contain one or more oils selected from the group consisting of a hydrocarbon oil, an ether oil, and a silicone oil in addition to the ester oil other than the component (B1), from the viewpoint of use impression such as smoothness upon application.

Examples of the ester oil other than the component (B1) include ester of linear or branched fatty acid and linear or branched alcohol or polyhydric alcohol. Specific examples thereof include isopropyl myristate, cetyl octanoate, octyldodecyl myristate, isopropyl palmitate, butyl stearate, hexyl laurate, myristyl myristate, decyl oleate, hexyldecyl dimethyloctanoate, isotridecyl isononanoate, isononyl isononanoate, dicaprylyl carbonate, propylheptyl caprylate, diisopropyl sebacate, triethylhexanoin, cetyl lactate, myristyl lactate, dicaprylyl carbonate, lanoline acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimehylolpropane tri-2-ethylhexanoate, trimethylolpropane triisostearate, pentaerythritol tetra-2-ethylhexanoate, glyceryl tri-2-ethylhexanoate, trimethylolpropane triisostearate, cetyl 2-ethylhexanoate, 2-ethylhexyl palmitate, diethylhexyl naphthalenedicarboxylate, C12-15 alkyl benzoate, cetearyl isononanoate, glycerin tri(caprylate-caprate), butylene glycol (dicaprylate/caprate), glyceryl trilaurate, glyceryl trimyristate, glyceryl tripalmitate, glyceryl triisostearate, glyceryl tri-2-heptylundecanoate, glyceryl tribehenate, tri-coconut oil fatty acid glyceryl, castor oil fatty acid methyl ester, oleyl oleate, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di-2-ethylhexyl succinate, triethyl citrate, and tripropylene glycol dipivalate.

The ester oil other than the component (B1) more preferably is one or more members selected from the group consisting of isopropyl myristate, isopropyl palmitate, neopentyl glycol dicaprate, pentaerythritol tetra-2-ethylhexanoate, glycerin tri(caprylate-caprate), butylene glycol (dicaprylate/caprate), 2-hexyldecyl myristate, isotridecyl isononanoate, isononyl isononanoate, dicaprylyl carbonate, propylheptyl caprylate, diisopropyl sebacate, and triethylhexanoin from the viewpoint of dissolving the water-insoluble ultraviolet absorber which is in a solid state at 25°C. A vegetable oil or an animal oil containing such an ester oil may be used. For example, olive oil, jojoba oil, macadamia nut oil, meadowfoam oil, castor oil, safflower oil, sunflower oil, avocado oil, canola oil, apricot kernel oil, rice germ oil, or rice bran oil can be used.

Examples of the hydrocarbon oil include liquid paraffin, squalane, squalene, n-octane, n-heptane, cyclohexane, light isoparaffin, and liquid isoparaffin. Liquid paraffin and squalane are preferred from the viewpoint of use impression.

Examples of the ether oil include cetyl-1,3-dimethyl butyl ether, dicapryl ether, dilauryl ether, and diisostearyl ether.

Examples of the silicone oil include dimethylpolysiloxane, methylcyclopolysiloxane, methylphenylpolysiloxane, methyl hydrogen polysiloxane, and higher alcohol-modified organopolysiloxane.

Examples of the fluorine oil include fluoropolyether and perfluoroalkyl ether silicone.

The content of the component (B) is preferably 12% by mass or more, more preferably 12.5% by mass or more, further more preferably 13% by mass or more, in the whole amount of the sunscreen cosmetic of the present invention from the viewpoint of dissolving the component (A) and reducing feel upon application and stickiness. The component (B) is contained at preferably 40% by mass or less, more preferably 35% by mass or less, further more preferably less than 30% by mass, even more preferably less than 24% by mass, from the same viewpoint as above. Specifically, the content is preferably 12% by mass or more and 40% by mass or less, more preferably 12.5% by mass or more and 35% by mass or less, further more preferably 13% by mass or more and less than 30% by mass, even more preferably 13% by mass or more and less than 24% by mass. In the present invention, even if the content of the component (B) is less than 30% by mass or further less than 24% by mass, the component (A) can be sufficiently dissolved and feel and stickiness after application can be reduced.

The water-insoluble organic ultraviolet absorber (B1) which is in a liquid state at 25°C is contained at preferably 1% by mass or more, more preferably 2% by mass or more, further more preferably 3% by mass or more, in the whole amount of the sunscreen cosmetic of the present invention from the viewpoint of producing a better ultraviolet protection effect while dissolving the component (A). The component (B1) is contained at preferably 8% by mass or less, more preferably 7% by mass or less, further more preferably 5% by mass or less, from the same viewpoint as above. Specifically, the content is preferably 1% by mass or more and 8% by mass or less, more preferably 2% by mass or more and 7% by mass or less, further more preferably 3% by mass or more and 5% by mass or less.

The content of the water-insoluble ultraviolet absorber (B1) which is in a liquid state at 25°C is preferably smaller than that of the component (A) from the viewpoint of producing feel upon application, specifically, non-sticky feeling and cool feeling. The mass ratio of the component (B1) to the component (A), (B1)/(A), is preferably less than 1, more preferably 0.7 or less, further more preferably 0.5 or less. The mass ratio (B1)/(A) is preferably 0.1 or more, more preferably 0.2 or more, further more preferably 0.3 or more.

The mass ratio of the component (B) to the total amount of the component (A1) and the component (A2), (B)/((A1)+(A2)), is preferably 2.7 or more and 9 or less from the viewpoint of dissolving the component (A1) and the component (A2), suppressing crystallization, giving favorable feel upon application, and producing an excellent ultraviolet protection effect. The mass ratio is preferably 2.8 or more, more preferably 2.9 or more, further more preferably 3.0 or more, and preferably 8.7 or less, more preferably 8.5 or less, further more preferably 8.3 or less, from the same viewpoint as above. Specifically, the mass ratio is preferably 2.8 or more and 8.7 or less, more preferably 2.9 or more and 8.5 or less, further more preferably 3.0 or more and 8.3 or less.

The mass ratio of the component (B) to the component (A), (B)/(A), is preferably 1 or more and 3 or less, more preferably 1 or more and 2.2 or less, further more preferably 1 or more and 2 or less, from the viewpoint of use impression.

The component (C) is ethanol.

The ethanol is contained at preferably 48% by mass or more, more preferably 49% by mass or more, further more preferably 49.5% by mass or more, in the whole amount of the sunscreen cosmetic of the present invention from the viewpoint of thoroughly dissolving the components (A) and (B) to prepare a stable sunscreen cosmetic, and attaining cool and non-sticky feel upon application. The ethanol is contained at preferably 78% by mass or less, more preferably 77% by mass or less, further more preferably 75% by mass or less, from the same viewpoint as above. Specifically, the content is preferably 48% by mass or more and 78% by mass or less, more preferably 49% by mass or more and 77% by mass or less, further more preferably 49.5% by mass or more and 75% by mass or less.

The mass ratio of the component (C) to the total amount of the component (A) and the component (B), (C)/((A)+(B)), is preferably 0.9 or more and 3.0 or less, more preferably 1.5 or more and 3.0 or less, from the viewpoint of use impression such as sticky feeling and cool feeling.

The effect of the ethanol is particularly remarkably exhibited when the sunscreen cosmetic of the present invention is in a form filled into a jet container.

The sunscreen cosmetic of the present invention can contain, in addition to the components described above, an ultraviolet scattering agent, an oil gelling agent, a surfactant, a water-soluble polymer, a neutralizing agent, a germicide, an anti-inflammatory agent, an antiseptic, a colorant, a chelating agent, a skin lightening agent, an antiperspirant, a refreshing agent, an insect repellent, a biologically active component, an antioxidant, a fragrance, and the like.

The sunscreen cosmetic of the present invention contains the components (A) and (B) dissolved in ethanol as described above. Therefore, the content of water is preferably 10% by mass or less, more preferably 5% by mass or less. A mass ratio of water/ethanol is preferably 0 or more and less than 0.25, more preferably 0.01 or more and less than 0.15, further more preferably 0.02 or more and less than 0.1.

The content of the surfactant in the sunscreen cosmetic of the present invention is preferably less than 1% by mass, more preferably less than 0.5% by mass, further more preferably 0% by mass, from the viewpoint of feel upon application.

The sunscreen cosmetic of the present invention may be in the form of a liquid composition containing the components described above and is more preferably in the form of a sunscreen cosmetic prepared by filling the liquid composition into a jet container.

When the sunscreen cosmetic of the present invention is a liquid composition, for example, the liquid composition filled into a container can be taken with hands and applied to the skin.

The jet may eject oil droplets or may eject mist. Ejecting mist is preferable from the viewpoint of feel upon application.

The jet container includes an aerosol container and a spray container with pump.

A preparation of the composition filled into an aerosol container (aerosol preparation) contains the composition and a propellant. The aerosol preparation containing the sunscreen cosmetic composition of the present invention (hereinafter, also referred to as the composition) can confer cool feeling or the like by jetting and applying the composition to the skin. In the aerosol preparation, the composition for use as an aerosol undiluted solution and its suitable aspect are the same as described above. Specifically, the contents of the components in the aerosol stock solution preferably fall within the ranges described above.

Examples of the propellant for use in the aerosol preparation include ethane, propane, normal butane, isobutane, isopentane, and liquefied petroleum gas (LPG) which is a mixture thereof; ethers such as dimethyl ether; and compressed gases such as nitrogen and carbon dioxide, one or more of which can be used.

In the aerosol preparation, the quantitative ratio between the composition serving as an aerosol stock solution and the propellant is not particularly limited as long as the composition can be jetted to the skin. The mass ratio between the composition and the propellant is in the range of preferably from 1 : 0.01 to 1 : 10, more preferably from 1 : 0.05 to 1 : 7.5, from the viewpoint of aerosol performance and the stability of the component (A).

When the propellant is LPG, the mass ratio between the composition and the LPG is further more preferably from 1 : 0.5 to 1 : 5. When the propellant is dimethyl ether, the mass ratio between the composition and the dimethyl ether is further more preferably from 1 : 0.1 to 1 : 5. When the propellant is carbon dioxide, the mass ratio between the composition and the carbon dioxide is further more preferably from 1 : 0.1 to 1 : 0.5.

Examples of the aerosol container for use in the aerosol preparation include known pressure-resistant containers made of metals or plastics, and double-structure containers in which an inner bag is housed inside the pressure-resistant container. In the double-structure container, it is preferred to fill the composition serving as an aerosol stock solution into the inner bag and fill the propellant to between the pressure-resistant container and the inner bag.

A method for preparing the aerosol preparation is not particularly limited. The aerosol preparation can be prepared, for example, by filling the composition serving as an aerosol stock solution into the aerosol container to install a valve, and subsequently filling the propellant thereinto from the valve.

The preparation prepared by filling the composition into a spray container with pump is also referred to as a pump spray-type preparation. For the pump spray-type preparation, for example, all the components except ethanol are mixed and dissolved by heating at 80°C. After confirmation of dissolution, the mixture is cooled to room temperature and mixed with ethanol to give a stock solution. The obtained stock solution can be filled into the spray container with pump.

One aspect of the present invention relates to a method for using a sunscreen cosmetic, comprising ejecting liquid droplets or mist of the composition from the jet container and applying the cosmetic to the skin.

The cosmetic of the present invention thus used produces an excellent ultraviolet protection effect, gives favorable feel upon application, and suppresses crystallization of the water-insoluble ultraviolet absorber which is in a solid state at 25°C.

In relation to the embodiments mentioned above, the present invention further discloses the following embodiments.
<1> A sunscreen cosmetic comprising the following components (A), (B), and (C), wherein a mass ratio of a component (A1) to a component (A2), (A1)/(A2), is 3.5 or more and 120 or less, a mass ratio of the component (B) to a total amount of the component (A1) and the component (A2), (B)/((A1)+(A2)), is 2.7 or more and 9 or less, and a mass ratio of a component (B1) to the component (A), (B1)/(A), is less than 1:
   (A) 10% by mass or more and 20% by mass or less of a water-insoluble organic ultraviolet absorber which is in a solid state at 25°C, the water-insoluble organic ultraviolet absorber comprising (A1) ethylhexyl triazone and (A2) bis-ethylhexyloxyphenol methoxy triazine,
   (B) 12% by mass or more and 40% by mass or less of an oil agent which is in a liquid state at 25°C, the oil agent comprising (B1) a water-insoluble organic ultraviolet absorber which is in a liquid state at 25°C, and
   (C) 48% by mass or more and 78% by mass or less of ethanol.
<2> The cosmetic according to <1>, wherein the component (A) further comprises one or more members selected from the group consisting of 4-tert-butyl-4'-methoxydibenzoylmethane, diethylamino hydroxybenzoyl hexyl benzoate, drometrizole trisiloxane, and ethylhexyl dimethoxybenzylidene dioxoimidazolidine propionate.
<3> The cosmetic according to <1> or <2>, wherein a content of the component (A1) is preferably 1.5% by mass or more and 10% by mass or less, more preferably 1.8% by mass or more and 9% by mass or less, further more preferably 2% by mass or more and 7% by mass or less, in the whole amount of the sunscreen cosmetic.
<4> The cosmetic according to any of <1> to <3>, wherein a content of the component (A2) is preferably 0.01% by mass or more and 1% by mass or less, more preferably 0.02% by mass or more and 0.9% by mass or less, further more preferably 0.05% by mass or more and 0.8% by mass or less, in the whole amount of the sunscreen cosmetic.
<5> The cosmetic according to any of <1> to <4>, wherein the mass ratio of the component (A1) to the component (A2), (A1)/(A2), is preferably 3.6 or more and less than 100, more preferably 3.6 or more and 50 or less, further more preferably 3.7 or more and 10 or less.
<6> The cosmetic according to any of <1> to <5>, wherein a total content of 2-ethylhexyl p-methoxycinnamate, oxybenzone, and octocrylene is 1% by mass or less.
<7> The cosmetic according to any of <1> to <6>, wherein the component (B1) is one or more ultraviolet absorbers which are in a liquid state at 25°C, the ultraviolet absorbers being selected from the group consisting of 2-ethoxyethyl p-methoxycinnamate, an isopropyl p-methoxycinnamate-diisopropyl cinnamic acid ester mixture, methyl bis(trimethylsiloxy)silylisopentyl trimethoxycinnamate, diethylamino hydroxybenzoyl hexyl benzoate, amyl p-dimethylaminobenzoate, 2-ethylhexyl p-dimethylaminobenzoate, ethylene glycol salicylate, 2-ethylhexyl salicylate, butyloctyl salicylate, benzyl salicylate, and homomenthyl salicylate.
<8> The cosmetic according to any of <1> to <7>, wherein the component (B) is an oil agent which is in a liquid state at 25°C, the oil agent comprising an ester oil other than the component (B1).
<9> The cosmetic according to <8>, wherein the component (B) further comprises one or more oil agents which are in a liquid state at 25°C, the oil agents being selected from the group consisting of a hydrocarbon oil, an ether oil, a silicone oil, and a fluorine oil.
<10> The cosmetic according to any of <1> to <9>, wherein a content of the component (B) is preferably 12.5% by mass or more and 35% by mass or less, more preferably 13% by mass or more and less than 30% by mass, in the whole amount of the sunscreen cosmetic.
<11> The cosmetic according to any of <1> to <10>, wherein a content of the component (B1) is preferably 1% by mass or more and 8% by mass or less, more preferably 2% by mass or more and 7% by mass or less, further more preferably 3% by mass or more and 5% by mass or less, in the whole amount of the sunscreen cosmetic.
<12> The cosmetic according to any of <1> to <11>, wherein the mass ratio of the component (B1) to the component (A), (B1)/(A), is preferably less than 1, more preferably 0.7 or less, further more preferably 0.5 or less, and preferably 0.1 or more, more preferably 0.2 or more, further more preferably 0.3 or more.
<13> The cosmetic according to any of <1> to <12>, wherein the mass ratio of the component (B) to the total amount of the component (A1) and the component (A2), (B)/((A1)+(A2)), is preferably 2.7 or more and 8.7 or less, more preferably 2.8 or more and 8.5 or less, further more preferably 3.0 or more and 8.3 or less.
<14> The cosmetic according to any of <1> to <13>, wherein the mass ratio of the component (B) to the component (A), (B)/(A), is preferably 1 or more and 3 or less, more preferably 1 or more and 2.2 or less, further more preferably 1 or more and 2 or less.
<15> The cosmetic according to any of <1> to <14>, wherein a content of the ethanol is preferably 49% by mass or more and 77% by mass or less, more preferably 49.5% by mass or more and 75% by mass or less.
<16> The cosmetic according to any of <1> to <15>, wherein a mass ratio of the component (C) to the total amount of the component (A) and the component (B), (C)/((A)+(B)), is preferably 0.9 or more and 3.0 or less, more preferably 1.5 or more and 3.0 or less.
<17> The cosmetic according to any of <1> to <16>, wherein a content of water is 10% by mass or less.
<18> A sunscreen cosmetic prepared by filling a composition according to any of <1> to <17> into a jet container.
<19> The cosmetic according to <18>, wherein the jet container is an aerosol container or a spray container with pump.
<20> A sunscreen cosmetic which is used by filling a composition according to any of <1> to <17> into a jet container.
<21> The cosmetic according to <20>, wherein the jet container is an aerosol container or a spray container with pump.
<22> A method for using a sunscreen cosmetic, comprising ejecting liquid droplets or mist of a cosmetic according to any of <18> to <21> from the jet container and applying the cosmetic to the skin.

### Examples

Next, the present invention will be described in more detail with reference to Examples. However, the present invention is not limited by these Examples.

### [Production of sunscreen cosmetic]

All components except for ethanol described in Tables 1 to 3 were mixed and dissolved by heating at 80°C. After confirmation of dissolution, the mixture was cooled to room temperature and mixed with ethanol to give a stock solution. The obtained stock solution was filled into a spray container with pump, to produce sunscreen cosmetics.

### [Evaluation of sunscreen cosmetic]

The pump spray-type sunscreen cosmetics thus obtained or their stock solutions were used to evaluate (1) an ultraviolet protection effect, (2) an effect of suppressing the crystallization of the components (A1) and (A2), (3) sticky feeling, and (4) feel immediately after application. The obtained results are shown in Tables 1 to 3.

### (1) Ultraviolet protection effect

Each composition (stock solution) was applied at 2 mg/cm² to a PMMA plate, and vitro SPF was measured with an SPF analyzer.
A: Vitro SPF of 30 or more
B: Vitro SPF of 20 or more and less than 30
C: Vitro SPF of 10 or more and less than 20
D: Vitro SPF of 10 or less

### (2) Crystallization-suppressing effect

Each composition (stock solution) was preserved at - 5°C and evaluated for crystallization.
A: Crystallization was not observed during 6-month preservation.
B: Crystallization was not observed during 2-week preservation.
C: Slight crystallization was observed during 2-week preservation.
D: Crystallization was observed during 2-week preservation.

### (3) Sticky feeling

Each composition (stock solution) was filled into a spray container with pump. Each of five specialized panelists sprayed 0.4 g thereof to one arm and assessed sticky feeling immediately after application on the basis of criteria given below. The total score of the five panelists was used in evaluation.
1: Sticky
2: Slightly sticky
3: Barely sticky
4: Non-sticky

### (Evaluation of total score)

A: Total score was 15 or more
B: Total score was 10 or more and less than 15
C: Total score was 5 or more and less than 10
D: Total score was less than 5

### (4) Cool feeling

Each composition (stock solution) was filled into a spray container with pump. Each of five specialized panelists sprayed 0.4 g thereof to one arm and assessed cool feeling immediately after application on the basis of criteria given below. The total score of the five panelists was used in evaluation.
1: Not cool
2: Barely cool
3: Cool
4: Very cool

### (Evaluation of total score)

A: Total score was 15 or more
B: Total score was 10 or more and less than 15
C: Total score was 5 or more and less than 10
D: Total score was less than 5

**[Table 1]**

| | Component name | Example 1 | Example 2 | Example 3 | Example 4 | Example 5 | Example 6 | Example 7 | Example 8 | Example 9 |
|---|---|---|---|---|---|---|---|---|---|---|
| Component (A) | (A1) Ethvlhexvl triazone | 4.0 | 3.5 | 3.0 | 2.5 | 3.0 | 3.0 | 5.0 | 4.0 | 3.5 |
| | (A2) Bis-ethylhexyloxyphenol methoxyphenyl triazine | 0.6 | 0.6 | 0.6 | 0.6 | 0.8 | 0.7 | 0.05 | 0.7 | 0.7 |
| | Diethylamino hydroxybenzoyl hexyl benzoate | 5.0 | 5.0 | 5.0 | 5.0 | 5.0 | 5.0 | 5.0 | 5.0 | 5.0 |
| | Drometrizole trisiloxane | 3.0 | 3.0 | 3.0 | 3.0 | 3.0 | 3.0 | 3.0 | 3.0 | 3.0 |
| Component (B) | (B1) Ethylhexyl salicylate | 4.85 | 4.85 | 4.85 | 4.85 | 4.85 | 4.85 | 4.85 | 4.85 | 4.85 |
| | C12-15 Alkyl benzoate | 3.0 | 3.0 | 3.0 | 3.0 | 3.0 | 3.0 | 3.0 | 3.0 | 3.0 |
| | Dicaprylyl carbonate | 7.0 | 7.0 | 7.0 | 7.0 | 7.0 | 7.0 | 7.0 | 7.0 | 7.0 |
| Component (C) | Ethanol | 72.55 | 73.05 | 73.55 | 74.05 | 73.35 | 73.45 | 72.10 | 72.45 | 72.95 |
| Total | | 100 | 100 | 100 | 100 | 100 | 100 | 100 | 100 | 100 |
| (A1)/(A2) | | 6.67 | 5.83 | 5.00 | 4.17 | 3.75 | 4.29 | 100 | 5.71 | 5.00 |
| (B)/((A1)+(A2)) | | 3.23 | 3.62 | 4.13 | 4.79 | 3.91 | 4.01 | 2.94 | 3.16 | 3.54 |
| (B1)/(A) | | 0.38 | 0.40 | 0.42 | 0.44 | 0.41 | 0.41 | 0.37 | 0.38 | 0.40 |
| Content of component (A) | | 12.6 | 12.1 | 11.6 | 11.1 | 11.8 | 11.7 | 13.1 | 12.7 | 12.2 |
| Content of component (B) | | 14.85 | 14.85 | 14.85 | 14.85 | 14.85 | 14.85 | 14.85 | 14.85 | 14.85 |
| B/A | | 1.18 | 1.23 | 1.28 | 1.34 | 1.26 | 1.27 | 1.14 | 1.17 | 1.22 |
| (C)/((A)+(B)) | | 2.64 | 2.71 | 2.78 | 2.85 | 2.75 | 2.77 | 2.58 | 2.63 | 2.70 |
| Crystallization suppressing effect | | A | A | B | B | B | B | A | A | B |
| Sticky feeling | | A | A | A | A | A | A | A | A | A |
| Cool feeling | | A | A | A | A | A | A | A | A | A |
| Ultraviolet protection effect | | A | A | A | A | A | A | B | A | A |

**[Table 2]**

| | Component name | Example 10 | Example 11 | Example 12 | Example 13 | Example 14 | Example 15 | Example 16 | Example 17 | Example 18 |
|---|---|---|---|---|---|---|---|---|---|---|
| Component (A) | (A1) Ethylhexyl triazone | 4.0 | 4.0 | 4.0 | 4.0 | 4.0 | 4.0 | 4.0 | 4.0 | 4.0 |
| | (A2) Bis-ethylhexyloxyphenol methoxyphenyl triazine | 0.6 | 0.6 | 0.6 | 0.6 | 0.6 | 0.6 | 0.6 | 0.6 | 0.6 |
| | Diethylamino hydroxybenzoyl hexyl benzoate | 5.0 | 5.0 | 5.0 | 5.0 | 5.0 | 5.0 | 5.0 | 5.0 | 5.0 |
| | Drometrizole trisiloxane | 3.0 | 3.0 | 3.0 | 3.0 | 3.0 | 3.0 | 3.0 | 3.0 | 3.0 |
| Component (B) | (B1) Ethylhexyl salicylate | 4.85 | 4.85 | 4.85 | 4.85 | 4.85 | 4.85 | 4.85 | 4.85 | 4.85 |
| | C12-15 Alkyl benzoate | 2.0 | 1.0 | 3.0 | 3.0 | 3.0 | 3.0 | 3.0 | 3.0 | 3.0 |
| | Dicaprylyl carbonate | 7.0 | 7.0 | 6.0 | 5.0 | 10 | 15 | 20 | 25 | 30 |
| Component (C) | Ethanol | 73.55 | 74.55 | 73.55 | 74.55 | 69.55 | 64.55 | 59.55 | 54.55 | 49.55 |
| Total | | 100 | 100 | 100 | 100 | 100 | 100 | 100 | 100 | 100 |
| (A1)/(A2) | | 6.67 | 6.67 | 6.67 | 6.67 | 6.67 | 6.67 | 6.67 | 6.67 | 6.67 |
| (B)/((A1)+(A2)) | | 3.01 | 2.79 | 3.01 | 2.79 | 3.88 | 4.97 | 6.05 | 7.14 | 8.23 |
| (B1)/(A) | | 0.38 | 0.38 | 0.38 | 0.38 | 0.38 | 0.38 | 0.38 | 0.38 | 0.38 |
| Content of component (A) | | 12.6 | 12.6 | 12.6 | 12.6 | 12.6 | 12.6 | 12.6 | 12.6 | 12.6 |
| Content of component (B) | | 13.85 | 12.85 | 13.85 | 12.85 | 17.85 | 22.85 | 27.85 | 32.85 | 37.85 |
| (B)/(A) | | 1.10 | 1.02 | 1.10 | 1.02 | 1.42 | 1.81 | 2.21 | 2.61 | 3.00 |
| (C)/((A)+(B)) | | 2.78 | 2.93 | 2.78 | 2.93 | 2.28 | 1.82 | 1.47 | 1.20 | 0.98 |
| Crystallization suppressing effect | | A | B | A | B | A | A | A | A | A |
| Sticky feeling | | A | A | A | A | A | A | B | B | B |
| Cool feeling | | A | A | A | A | A | A | B | B | B |
| Ultraviolet protection effect | | A | A | A | A | A | A | A | A | A |

**[Table 3]**

| | Component name | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|
| Component (A) | (A1) Ethylhexyl triazone | | 4.0 | 3.0 | 3.0 | 4.0 | 4.0 | 4.0 | 4.0 | 5.0 | 4.0 |
| | (A2) Bis-ethylhexyloxyphenol methoxyphenyl triazine | 0.6 | | 1.0 | 0.9 | 0.6 | 0.6 | 0.6 | 0.6 | 0.6 | 0.6 |
| | Diethylamino hydroxybenzoyl hexyl benzoate | 5.0 | 5.0 | 5.0 | 5.0 | 5.0 | 5.0 | 5.0 | 5.0 | 10.0 | 5.0 |
| | Drometrizole trisiloxane | 3.0 | 3.0 | 3.0 | 3.0 | 3.0 | 3.0 | 3.0 | 3.0 | 4.4 | 3.0 |
| Component (B) | (B1) Ethylhexyl salicylate | 4.85 | 4.85 | 4.85 | 4.85 | 4.85 | | 4.85 | 4.85 | 4.85 | 15 |
| | C12-15 Alkyl benzoate | 3.0 | 3.0 | 3.0 | 3.0 | 3.0 | 3.0 | | 3.0 | 5.0 | 3.0 |
| | Dicaprylyl carbonate | 7.0 | 7.0 | 7.0 | 7.0 | 35.0 | 7.0 | 7.0 | | 30.15 | 7.00 |
| Component (C) | Ethanol | 76.55 | 73.15 | 73.15 | 73.25 | 44.55 | 77.40 | 75.55 | 79.55 | 40.00 | 62.40 |
| Total | | 100 | 100 | 100 | 100 | 100 | 100 | 100 | 100 | 100 | 100 |
| (A1)/(A2) | | 0.00 | | 3.00 | 3.33 | 6.67 | 6.67 | 6.67 | 6.67 | 8.33 | 6.67 |
| (B)/((A1)+(A2)) | | 24.75 | 3.71 | 3.71 | 3.81 | 9.32 | 2.17 | 2.58 | 1.71 | 7.14 | 5.43 |
| (B1)/(A) | | 0.56 | 0.40 | 0.40 | 0.41 | 0.38 | 0.00 | 0.38 | 0.38 | 0.24 | 1.19 |
| Content of component (A) | | 8.6 | 12.0 | 12.0 | 11.9 | 12.6 | 12.6 | 12.6 | 12.6 | 20.0 | 12.6 |
| Content of component (B) | | 14.85 | 14.85 | 14.85 | 14.85 | 42.85 | 10.00 | 11.85 | 7.85 | 40.00 | 25.00 |
| (B)/(A) | | 1.73 | 1.24 | 1.24 | 1.25 | 3.40 | 0.79 | 0.94 | 0.62 | 2.00 | 1.98 |
| (C)/((A)+(B)) | | 3.26 | 2.72 | 2.72 | 2.74 | 0.80 | 3.42 | 3.09 | 3.89 | 0.67 | 1.66 |
| Crystallization suppressing effect | | D | A | D | D | A | D | D | D | A | A |
| Sticky feeling | | A | A | B | A | C | A | A | A | C | C |
| Cool feeling | | A | A | A | A | B | A | A | A | C | B |
| Ultraviolet protection effect | | D | C | A | A | A | B | B | B | A | A |

## Claims

1. A sunscreen cosmetic comprising the following components (A), (B), and (C), wherein a mass ratio of a component (A1) to a component (A2), (A1)/(A2), is 3.5 or more and 120 or less, a mass ratio of the component (B) to a total amount of the component (A1) and the component (A2), (B)/((A1)+(A2)), is 2.7 or more and 9 or less, and a mass ratio of a component (B1) to the component (A), (B1)/(A), is less than 1:
(A) 10% by mass or more and 20% by mass or less of a water-insoluble organic ultraviolet absorber which is in a solid state at 25°C, the water-insoluble organic ultraviolet absorber comprising (A1) ethylhexyl triazone and (A2) bis-ethylhexyloxyphenol methoxy triazine,
(B) 12% by mass or more and 40% by mass or less of an oil agent which is in a liquid state at 25°C, the oil agent comprising (B1) a water-insoluble organic ultraviolet absorber which is in a liquid state at 25°C, and
(C) 48% by mass or more and 78% by mass or less of ethanol.

2. The sunscreen cosmetic according to claim 1, wherein the component (B) is an oil agent which is in a liquid state at 25°C, the oil agent comprising an ester oil other than the component (B1).

3. The sunscreen cosmetic according to claim 2, wherein the component (B) further comprises one or more oil agents which are in a liquid state at 25°C, the oil agents being selected from the group consisting of a hydrocarbon oil, an ether oil, a silicone oil, and a fluorine oil.

4. The sunscreen cosmetic according to any one of claims 1 to 3, wherein the component (B1) comprises one or more ultraviolet absorbers which are in a liquid state at 25°C, the ultraviolet absorbers being selected from the group consisting of 2-ethoxyethyl p-methoxycinnamate, an isopropyl p-methoxycinnamate-diisopropyl cinnamic acid ester mixture, methyl bis(trimethylsiloxy)silylisopentyl trimethoxycinnamate, diethylamino hydroxybenzoyl hexyl benzoate, amyl p-dimethylaminobenzoate, 2-ethylhexyl p-dimethylaminobenzoate, ethylene glycol salicylate, 2-ethylhexyl salicylate, butyloctyl salicylate, benzyl salicylate, and homomenthyl salicylate.

5. The sunscreen cosmetic according to any one of claims 1 to 4, wherein a content of the component (B) is 12% by mass or more and less than 30% by mass.

6. The sunscreen cosmetic according to any one of claims 1 to 5, wherein the component (A) further comprises one or more water-insoluble organic ultraviolet absorbers which are in a solid state at 25°C, the water-insoluble organic ultraviolet absorbers being selected from the group consisting of 4-tert-butyl-4'-methoxydibenzoylmethane, diethylamino hydroxybenzoyl hexyl benzoate, 2-hydroxy-4-methoxybenzophenone, and drometrizole trisiloxane.

7. The sunscreen cosmetic according to any one of claims 1 to 6, containing water in an amount of 10% by mass or less.

8. The sunscreen cosmetic according to any one of claims 1 to 7, wherein a total content of 2-ethylhexyl p-methoxycinnamate, oxybenzone, and octocrylene is 1% by mass or less.

9. A sunscreen cosmetic prepared by filling a composition according to any one of claims 1 to 8 into a jet container.

10. The cosmetic according to claim 9, wherein the jet container is an aerosol container or a spray container with pump.

11. A method for using a sunscreen cosmetic, comprising ejecting liquid droplets or mist of a cosmetic according to claim 9 or 10 from the jet container and applying the cosmetic to a skin.
